(19)

Europäisches
Patentamt
European
Patent Office
Office européen
des brevets

(11) **EP 3 446 725 A1**

(12) **EUROPEAN PATENT APPLICATION**

(43) Date of publication:
**27.02.2019 Bulletin 2019/09**

(21) Application number: **18189989.9**

(22) Date of filing: **21.08.2018**

(51) Int Cl.:
**A61L 29/12** *(2006.01)*        **A61L 29/08** *(2006.01)*
**A61F 2/958** *(2013.01)*        **A61M 25/10** *(2013.01)*
**B82Y 30/00** *(2011.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.08.2017   US 201762548570 P**

(71) Applicant: **Cook Medical Technologies LLC**
**Bloomington, IN 47404 (US)**

(72) Inventor: **MOELLER, Rasmus Buch**
**4100 Ringsted (DK)**

(74) Representative: **Garratt, Peter Douglas et al**
**Mathys & Squire LLP**
**The Shard**
**32 London Bridge Street**
**London SE1 9SG (GB)**

(54) **MEDICAL BALLOONS WITH NANOPLATELET COMPOSITE MATERIALS AND METHOD OF MAKING THE SAME**

(57)    A medical device includes an inflatable balloon having an axial length with a wall located along the axial length having an interior surface and an exterior surface that defines a wall thickness there between. At least a portion of the wall is formed of a nanoplatelet composite material that includes a polymeric matrix and one or more platelet nanofillers. The platelet nanofillers are aligned in a predetermined pattern. The predetermined pattern allows the platelet nanofillers to extend in at least two directions ranging from the nanofillers being circumferentially-oriented to axially-oriented relative to the axial length of the inflatable balloon.

Figure 1

**Description**

**FIELD**

This disclosure relates generally to medical balloons reinforced with nanoplatelet composite materials, the use of such reinforced medical balloons, and a method of fabricating the reinforced medical balloons.

**BACKGROUND**

The statements in this section merely provide background information related to the present disclosure and may not constitute prior art.

The body includes a variety of passageways in the form of arteries and other blood vessels. A passageway can sometimes become constricted or blocked by the accumulation of plaque or the growth of a tumor. When this occurs, the constricted passageway needs to be expanded in an angioplasty procedure using a balloon catheter, which includes a medical balloon carried on a catheter shaft.

Balloon angioplasty has become a widely used procedure for expanding constricted passageways. In the angioplasty procedure, a catheter delivers an uninflated medical balloon to the constricted region in the passageway. After positioning the balloon in the passageway, fluid injected through the catheter's lumen into the balloon causes the balloon to inflate and exert pressure against the constricted region to expand the passageway. After use, the balloon is collapsed and withdrawn with the catheter.

During use, medical balloons must expand in a predictable manner and withstand exposure to very high pressures that force the surface of the balloon against various vessel tissues and deposits that exhibit a variety of viscoelastic characteristics and may include some hard and/or rough surfaces. In this sense, the balloons must exhibit high strength and be puncture resistant. In addition, the balloons must also be thin-walled in order to collapse into a small cross-sectional profile necessary for introduction to and removal from the constricted passageway via a catheter.

**SUMMARY**

The present disclosure generally provides a medical device that comprises an inflatable balloon having an axial length with a wall located along the axial length having an interior surface and an exterior surface that defines a wall thickness there between; at least a portion of the wall being formed of a nanoplatelet composite material. The nanoplatelet composite material comprises a polymeric matrix and one or more platelet nanofillers; wherein the platelet nanofillers are dispersed and aligned in a predetermined pattern. The predetermined pattern comprises the platelet nanofilers extending in at least two directions ranging from the platelet nanofillers being circumferentially-oriented to axially-oriented relative to the axial length of the inflatable balloon.

According to another aspect of the present disclosure, a method of forming a medical device comprising an inflatable balloon having an axial length with a wall located along the axial length having an interior surface and an exterior surface that defines a wall thickness there between is provided. The method comprises providing a polymeric matrix; providing one or more platelet nanofillers; dispersing the one or more platelet nanofillers into the polymeric matrix to form a nanoplatelet composite material; forming the inflatable balloon from the nanoplatelet composite material using an extrusion process, a blow molding process, an injection molding process, a compression molding process, or a sheet-forming process; and aligning the platelet nanofiller in a predetermined pattern. The predetermined pattern comprises the platelet nanofilers extending in at least two directions ranging from the platelet nanofillers being circumferentially-oriented to axially-oriented relative to the axial length of the inflatable balloon circumferentially and/or axially with respect to the axial length of the inflatable balloon.

According to yet another aspect of the present disclosure, the use of a nanoplatelet composite material to form a medical device is provided wherein the medical device includes an inflatable balloon having an axial length with a wall located along the axial length having an interior surface and an exterior surface that defines a wall thickness there between. At least a portion of the wall is formed of the nanoplatelet composite material. The nanoplatelet composite material comprises a polymeric matrix; and one or more platelet nanofillers; wherein the platelet nanofillers are dispersed and aligned in a predetermined pattern. The predetermined pattern comprises the platelet nanofilers extending in at least two directions ranging from the platelet nanofillers being circumferentially-oriented to axially-oriented relative to the axial length of the inflatable balloon.

Further areas of applicability will become apparent from the description provided herein. It should be understood that the description and specific examples are intended for purposes of illustration only and are not intended to limit the scope of the present disclosure.

**DRAWINGS**

In order that the disclosure may be well understood, there will now be described various forms thereof, given by way of example, reference being made to the accompanying drawings, in which:

> Figure 1 is a perspective view of an inflatable balloon formed from a nanoplatelet composite material with platelet nanofillers aligned biaxially in a direction that is between axial-oreintation and circumferential-orientiation according to the teachings of the present disclosure;
> Figure 2 is a cross-sectional view of the inflatable balloon of Figure 1 taken along plane (a);
> Figure 3A a perspective view of a comparable inflatable balloon formed from a comparative nanocomposite material with platelet nanofillers aligned in a radial direction;
> Figure 3B is a cross-sectional view of the inflatable balloon of Figure 3A taken along plane (a);
> Figure 4A is a cross-sectional view of another inflatable balloon formed according to the teachings of the present disclosure that highlights the inclusion of protective layers;
> Figure 4B is a cross-sectional view of another inflatable balloon formed according to the teachings of the present disclosure in which the reinforced nanocomposite material is a coating applied the external surface of a polymer layer;
> Figure 5 is a flowchart describing a method of manufacturing a medical device having an inflatable medical balloon formed of a nanoplatelet composite material according to the teachings of the present disclosure; and
> Figure 6 is a schematic representation of various degrees of mixing or dispersing the platelet nanofiller in a polymeric matrix shown as a perspective 2-dimensional side-view (Note: the platelets would extend "inwards" into the paper from this view to provide a 3-dimensional perspective); and
> The drawings described herein are for illustration purposes only and are not intended to limit the scope of the present disclosure in any way.

**DETAILED DESCRIPTION**

The following description is merely exemplary in nature and is in no way intended, to limit the present disclosure or its application or uses. For example, the nanoplatelet composite materials made and used according to the teachings contained herein is described throughout the present disclosure in conjunction with thin tubular balloons used in angioplasty procedures performed in coronary vessels in order to more fully illustrate the composition and the use thereof. The incorporation and use of such nanoplatelet composite materials in medical balloons used in other applications, including valvuloplasty, minimally invasive repair of heart valves, the repair or removal of aneurysms, the deployment of stents in coronary and carotid arteries and other peripheral vessels, or the like are contemplated to be within the scope of the present disclosure. It should be understood that throughout the description, corresponding reference numerals indicate like or corresponding parts and features.

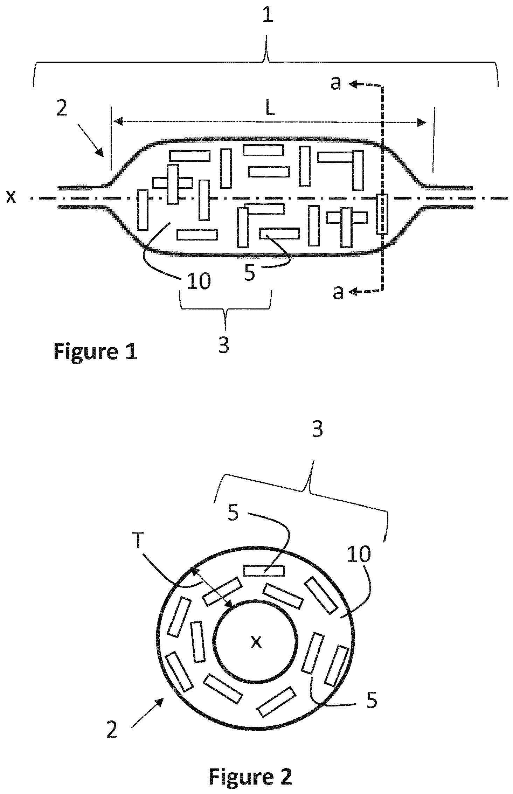

Referring to Figures 1, 2, & 3A, the present disclosure generally provides a medical device 1 that comprises an inflatable balloon 2 having a length (L) along axis (X) with a wall located along the axial length having an interior surface and an exterior surface that defines a wall thickness (T) there between. At least a portion of the wall is formed of a nanoplatelet composite 3. The nanoplatelet composite 3 comprises a polymeric matrix 10 and one or more platelet nanofillers 5, wherein the platelet nanofillers 5 are aligned in a predetermined pattern, wherein the predetermined pattern comprises the platelet nanofillers 5 extending biaxially in at least two directions ranging from the platelet nanofillers being circumferentially-oriented to axially-oriented relative to the axial length of the inflatable balloon

The ability to modify the properties of a medical balloon allows for customization of balloons for use in specific applications. The use of platelet nanofillers should enhance the strength of the balloon in both the axial and circumferential directions. Platelet nanofillers may also increase surface properties of the nanoplatelet composite materials with respect to scratch and/or puncture resistance. The modification of these properties can result in an increase or decrease in the targeted property as desired or necessary for use of an inflatable balloon in a predetermined application. For example, if a stronger more compliant balloon is desired, adding compliant platelet nanofillers to the polymeric matrix will increase tensile strength and elongation of the inflatable balloon. Similarly, if a stronger less compliant balloon is desired, the use of more rigid platelet nanofillers can increase the tensile strength and modulus, while decreasing elongation. Thus, the ability to modify the properties associated with the nanoplatelet composite materials allows for the use of unconventional materials in the formation of an inflatable balloon incorporated into a medical device.

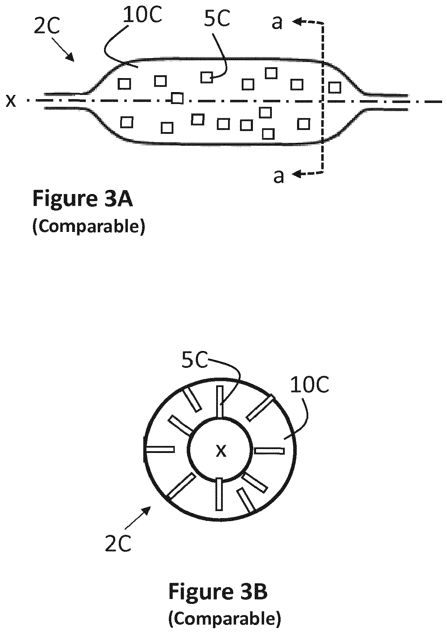

Referring once again to Figures 1, 2 & 3A, the platelet nanofillers 5 dispersed throughout the polymeric matrix 10 in the nanoplatelet composite materials 3 exhibit various lengths that are relatively linear in geometry. In other words, the platelet nanofillers 5 are aligned, such that a portion of them extends biaxially in two or more directions that range from the circumferential to axial directions relative to the axis (X) that corresponds to the axial length (L) of the inflatable balloon 2 in the medical device 1. Referring now to Figures 1 and 2, a portion of the platelet nanofillers 5 may be aligned in a circumferential direction relative to axis (X). Alternatively, a portion of the platelet nanofillers 5 are aligned in an axial direction relative to axis (X) or the axial length (L) of the inflatable balloon 2. Thus, the platelet nanofillers 5 therefore provide for the improvement of properties in directions that are circumferential or axial to the axis (X), as opposed to radial to axis (X). In other words, the inflatable balloon 2 may be compliant in the axial and circumferential directions and not in the other (radial) direction. Similarly, the strength exhibited by the inflatable balloon 2 in the two directions may be increased without doing so in the transverse or radial direction. A comparative example is provided in Figures 3A and 3B in which platelet nanofillers 5C dispersed in a polymeric matrix 10C are arranged in a radial direction relative to axis (X) or along the axial length (L) of an inflatable balloon 2C. Alignment of platelets in a radial direction is generally unlikely, and/or undesirable.

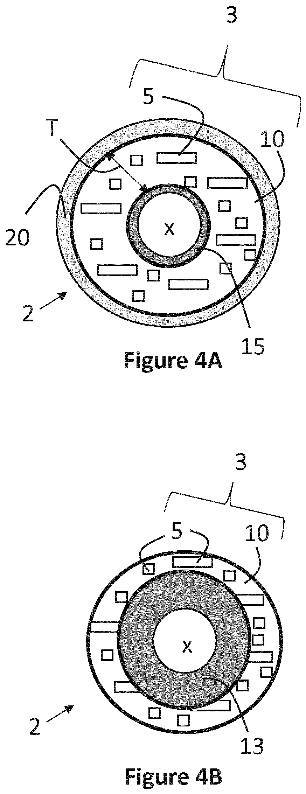

Referring now to Figure 4A, the nanoplatelet composite material 3 may also comprise at least one of an inner protective layer 25 and an outer protective layer 30. The protective layers 25, 30 do not need to be reinforced, but can be if so desired. These protective layers 25, 30 are individually selected to comprise the same material composition or a different composition than the polymeric matrix 10 of the nanoplatelet composite material 3.

According to another aspect of the present disclosure as shown in Figure 4B, the inflatable balloon 2 may comprise an inner layer of a polymeric material 13. In this case, the reinforced nanocomposite material 3 comprising the polymeric matrix 10 and the platelet nanofillers 5 is applied as a coating or outer layer onto the external surface of the inner polymer layer. The composition of the inner polymer layer may be the same or different from the composition of the polymeric matrix present in the reinforced nanocomposite material as further defined herein.

According to another aspect of the present disclosure, the orientation of the platelet nanofillers in the polymeric matrix of the nanoplatelet composite material of the inflatable medical balloon are arranged in a predetermined pattern. This predetermined pattern comprises the platelet nanofilers extending in at least two directions ranging from the platelet nanofillers being circumferentially-oriented to axially-oriented relative to the axial length of the inflatable balloon

When desirable or necessary to achieve a level of performance that is intermediate between the platelet nanocomposites with the platelet nanofillers axially and circumferentially oriented, a nanocomposite can be formed and used in which the platelet nanofillers are biaxially oriented at one or more angles that fall there between. The use of two or more layers to form the platelet nanocomposite materials can also be used to orient the nanofiller in each layer in a slightly different angle or direction within the confines of axial to circumferential orientation.

Medical balloons are required to perform under conditions wherein extensions greater than 100% are experienced. Upon stretching a medical balloon, the resulting extension in length is accompanied by a thinning of the cross-sectional area or wall thickness. Since the reinforcing platelet nanofillers usually are more rigid than the polymeric matrix, the reduction in wall thickness that occurs upon extension does not result in a proportional reduction in the thickness of the reinforcing nanofillers. Thus, the use of platelet nanofillers that are larger in size than the reduced wall thickness may cause distortions, exhibit reduced resistance to breakage, and provide points for potential failure. In other words, the use of reinforcing fillers in the nanoplatelet composite materials in the present disclosure is limited to platelet nanofillers.

As used herein the term "platelet nanofillers" is meant to include, but not be limited to layered inorganic fillers exhibiting a sheet structure with at least one dimension being in the nanometer range. Several specific examples of platelet nanofillers include, without limitation, clays, graphite, or the like, including organically-modified minerals. Each platelet nanofiller is individually selected, such that a mixture of different platelet nanofillers may be included in the nanoplatelet composite materials. The platelet nanofillers are incorporated into the polymeric matrix to form a nanoplatelet composite material in an amount that is between about 0.1 wt.% to less than 5 wt.%, based on the overall weight of the nanoplatelet composite material. Alternatively, the amount of platelet nanofillers incorporated into the nanoplatelet composite materials is less than about 3 wt.%, based on the overall weight of the nanoplatelet composite material. Alternatively, the nanoplatelet composite materials comprise between 0.1 wt. % to 4.0 wt.%, between 0.5 wt.% and 3.0 wt.%, from 1.0 wt.% to 3.0 wt.%, or from 1.0 wt.% to 2.0 wt.% platelet nanofillers, based on the overall weight of the nanoplatelet composite material.

For the purpose of this disclosure, the term "weight" refers to a mass value, such as having the units of grams, kilograms, and the like. Further, the recitations of numerical ranges by endpoints include the endpoints and all numbers within that numerical range. For example, a concentration ranging from 40% by weight (wt.%) to 60 wt.% by weight includes concentrations of 40% by weight, 60% by weight, and all concentrations there between (e.g., 40.1%, 41%, 45%, 50%, 52.5%, 55%, 59%, etc.).

The platelet nanofillers used in the nanoplatelet composite materials of the present disclosure may have a width and length that is in the range of about 0.5 nanometers to about 5 micrometers. Alternatively, the length and width of the platelet nanofillers may range from about 1 nanometers to 2 micrometers; alternatively, from about 10 nanometers to about 1 micrometer.

The platelet nanofillers have a 3rd dimension, namely, thickness. The thickness of the platelet nanofillers is usually less than the width or length of the platelet nanofillers. The thickness of the platelet nanofillers are in the range of about 5 Angstrom (A) to about 500 nanometers (nm); alternatively, from about 0.1 nm to about 100 nm; alternatively, in the range of about 0.5 nm to about 50 nm; alternatively, in the range of about 1 nm to about 10 nm.

According to another aspect of the present disclosure, the platelet nanofillers used in the nanoplatelet composite materials exhibit an aspect ratio of at least 25, at least 50, at least 100, or at least 250. Alternatively, the aspect ratio ranges from about 25 to about 10,000, from about 50 to about 5,000, from about 100 to about 1,000, or from about 200 to about 700. As used herein, the term "aspect ratio" refers to the ratio determined by dividing the average length or diagonal dimension of the platelet nanofillers by the average thickness of the platelet nanofillers as described in Equation 1.

$$\text{Aspect Ratio} = (\text{Length or Diagonal}) / \text{Thickness} \qquad \text{Eq. (1)}$$

The platelet nanofillers used in the present disclosure may be made of one or more natural or synthetic materials. According to one aspect of the present disclosure, the platelet nanofillers are biocompatible and comprise one or more synthetic or natural inorganic materials. Several examples of platelet nanofillers include but are not limited to graphite, graphene, minerals, and clays (including nanoclays). When desirable, the platelet nanofillers may organically-modified in order to enhance the compatability between the nanofiller and polymeric matrix. This organic modification may include without limitation the use of nanofibrilated cellulose (NFC) or cellulose nanocrystals (CNC). The platelet nanofillers may also comprise mica, bentonite, montmorillonite, hectorite, saponite, phyllosilicates, hydrotalcite, octasilicate, mica fluoride, or a combination thereof. A specific example of a nanoclay is CLOISITE® 30b (Southern Clay Products, Inc., Texas).

The platelet nanofillers may be inherently hydrophilic or hydrophobic, or the platelet nanofillers may be made to exhibit hydrophilic or hydrophobic properties by the addition of one or more surface functional groups. Several specific examples of hydrophilic functional groups include, without limitation, hydroxyl groups, carbonyl groups, carboxyl groups, and carboxylate groups. Several specific examples of hydrophobic functional groups include, but are not limited to hydrocarbons, silicones, and fluorocarbons.

Clays and nanoclays may include phyllosilicates, such as those more commonly found in the smectite group, for example a bentonite, montmorillonite, hectorite, saponite or the like. The surface of the clay can be modified to become organophilic hence clays are also sometimes referred to as organoclays. The inorganic exchangeable cations (e.g., potassium, sodium, etc.), which occur in natural and/or synthetic clay minerals, can be replaced by organic cations that comprise a sufficient number of carbon atoms that can render the surface of the cation-exchanged clay hydrophobic and organophilic. Several specific examples, among many examples, include the cationic exchange with cationic surfactants comprising alkyl ammonium-ions or functionalized organosilanes, in order to increase the spacing between the layers and reduces the surface energy of the nanofiller.

Graphite is a high-aspect-ratio, layered material that exhibits high electrical and thermal conductivities. Due to the occurrence of strong Van der Waals forces between the layers of graphite, it is difficult to separate the layers by simple mixing. However, it is possible to separate layers of graphite by chemical means. For example, exfoliated graphite may be prepared by intercalating layers of graphite with a strong acid (e.g., sulfuric or nitric acid) and then thermally decomposing the acid-intercalated graphite into separate sheets of exfoliated graphite.

The polymeric matrix in the nanoplatelet composite of the present disclosure may be made of one or more thermoset or thermoplastic polymers. The polymer may be a biocompatible polymer. Several specific examples of polymers used to form the polymeric matrix, include but are not limited to, a polyester, such as polyethylene terephthalate (PET), polybutylene terephthalate (PBT), polyethylene naphthalate (PEN), or ethylene terephthalate copolymers; a polyamide, such as nylon 6, 6/12, 11, 12, or 66; a polyurethane; a polyethylene; a polyvinyl chloride; a polycarbonate; a poly(meth)acrylate; a maleate; a polyether etherketone (PEEK); a poly(ethylene-co-methacrylic acid) (EMAA); a polyamide/polyether block copolymer; a polyester/polyether block copolymer; a polyamide/polyether polyester copolymer (e.g., Pebax®, Arkema, France); or a PBTpolybutylene oxide block copolymer. Alternatively, the polymeric matrix comprises a polyamide, such as nylon 6 or 12

When desirable other polymeric materials in the form of synthetic or natural rubbers may be used to form the polymeric matrix. These polymeric materials, may include but not be limited to, polyisoprene (synthetic natural rubber), polybutadiene, polychloroprene, butyl rubber, styrenebutadiene rubber, nitrile rubber, hydrogentated nitrile rubber (HNBR), ethylene propylene rubber, ethylene propylene diene rubber (EPDM), chlorosulphonated polyethylene (CSM), chlorinated polyethylene, polysulphide rubber, ethylene acrylic rubber, fluorocarbon rubber, polytetrafluoroethylene-propylene, epichlorohydrin rubber, polyacrylic rubber, silicone rubber, fluorosilicone rubber, polyphosphazene rubber, polyoctenylene, polypropylene oxide rubber, polynorbornene, polyether block amides, EVA rubber, styrenic block copolymers, or other segmented elastomers such as copolyester thermoplastic elastomers (TPEs).

The compatibility between the platelet nanofillers and the polymeric matrix may be enhanced by selecting platelet nanofillers and matrix materials that have compatible functional groups or by functionalizing the surface of the platelet nanofillers to incorporate such compatible groups that are capable of interacting with the matrix material. When desirable, one or more coupling or compatibilizing agents may also be incorporated into the nanoplatelet composite materials. As used herein, the term(s) coupling or compatibilizing agent includes any agent capable of enhancing the compatibility and/or promoting interaction between the platelet nanofillers and the polymeric matrix material. These compatibilizing agents may be organic or inorganic coupling agents. Several examples of organic coupling agents include, without limitation, amino acids, thiols, maleic anhydride containing polyolefins, and maleimide-functionalized polyamides. Several examples of inorganic coupling agents include, without limitation, alkoxides of silicon, aluminum, titanium, and zirconium, or magnetic powders such as ferrite oxide, to name a few. The amount of the coupling agent added to a nanoplatelet composite material may range from 0.1 wt.% to about 10 wt.% based on the weight of the nanocomposite material; alternatively, from about 0.5 wt.% to about 7.5 wt.%; alternatively, from about 1 wt. % to about 5 wt.% based on the weight of the nanoplatelet composite material.

The nanoplatelet composite materials may optionally comprise one or more other additives in the form of plasticizers, surfactants or dispersants, stabilizers, pigments, whiteners, conductive agents, magnetic agents, radiopaque agents, therapeutic agents (e.g., drugs), or a pharmaceutically-active compounds.

Inflatable medical balloons have a number of critical design parameters, such as rated burst pressure and tensile strength. The reinforcement of the polymeric matrix with the platelet nanofillers improves the strength and burst pressure of the medical balloon. The inflatable medical balloons of the present disclosure achieve a rated burst pressure (RBP) of at least 15 bar; alternatively, at least 20 bar; alternatively, at least 25 bar; alternatively about 30 bar or greater. The rated burst pressure (RBP) is the statistically determined maximum pressure to which a balloon may be inflated without rupturing. Burst testing of medical balloons may be accomplished using ISO 10555 methodology, according to FDA guidelines, or according to other standard industry protocols.

The medical balloons of the present disclosure may exhibit a tensile strength that is in the range of about 100 MPa to about 500 MPa; alternatively, in the range of about 200 MPa to about 400 MPa; alternatively, greater than 100 MPa; alternatively, greater than 200 MPa. The medical balloon may exhibit a maximum tensile strength up to 500 Mpa, up to 450 Mpa, up to 400 MPa, or alternatively, up to 350 Mpa. Tensile testing of medical balloons may be accomplished using ISO 10555 methodology, according to FDA guidelines, or according to other standard industry protocols.

In order to minimize the profile of the delivery system, it may be desirable that the balloon have a low wall thickness (T). However, a trade-off between burst pressure and wall thickness exists because the burst pressure generally decreases when the wall thickness is reduced. The inflatable medical balloons of the present disclosure have a wall thickness (T) that is less than 45 micrometers ($\mu$m), less than 35 $\mu$m, less than 25 $\mu$m, or less than 10 $\mu$m. Alternatively, the wall thickness (T) of the nanocomposite materials used to form the inflatable balloon ranges from about 5 micrometers to about 45 micrometers; alternatively, from about 10 micrometers to about 30 micrometers.

The inflatable balloons formed from the nanoplatelet composite materials of the present disclosure, which comprise platelet nanofillers dispersed and aligned in a polymeric matrix as described above and further defined herein, exhibit enhanced burst pressure and tensile strength as compared to inflatable balloons formed from a composite containing the same polymeric matrix in the absence of any platelet nanofillers. The enhancement in burst pressure and/or tensile strength exhibited by the inflatable balloons of the present disclosure are on the order of about 50% greater, about 25% greater, about 15% greater, about 10% greater, or alternatively, about 5% greater than the burst pressure and/or tensile strength exhibited by a similar balloon made of the same polymeric matrix in the absence of the platelet nanofillers.

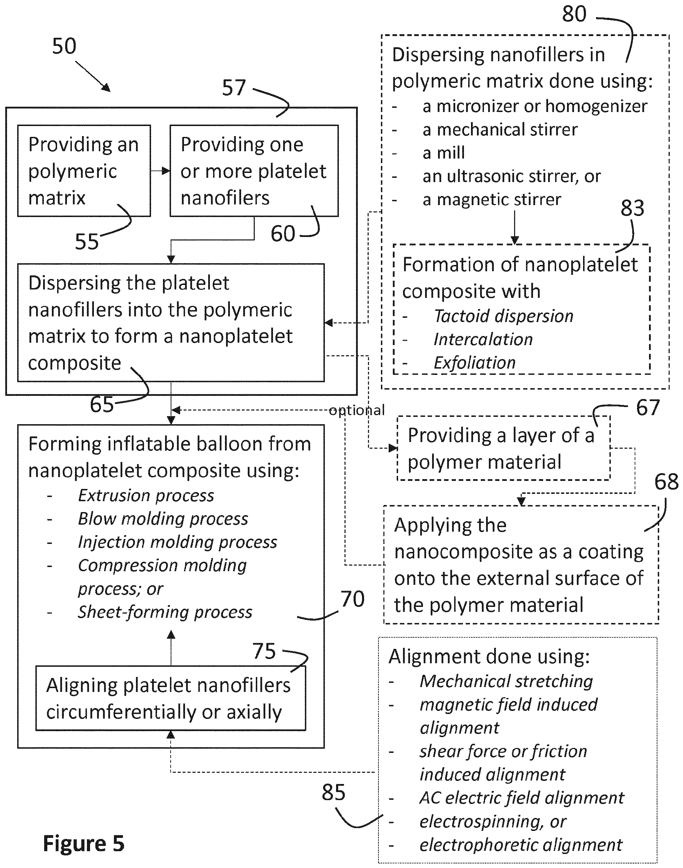

According to another aspect of the present disclosure, a method of forming a medical device comprising an inflatable balloon having an axial length with a wall located along the axial length having an interior surface and an exterior surface that defines a wall thickness there between is provided. Referring now to Figure 5, this method 50 generally comprises providing a polymeric matrix 55, providing one or more platelet nanofillers 60, followed by dispersing 65 the platelet nanofillers into the polymeric matrix to form a nanoplatelet composite material. The inflatable balloon is then formed 70 from the nanoplatelet composite material using an extrusion process, a blow molding process, an injection molding process, a compression molding process, a sheet-forming process, or the like. Then the platelet nanofillers are aligned 75 in a predetermined pattern. The predetermined pattern comprises the platelet nanofilers extending in at least two directions ranging from the platelet nanofillers being circumferentially-oriented to axially-oriented relative to the axial length of the inflatable balloon. The alignment 75 of the platelet nanofillers may be done as part of the forming process 70, for example during extrusion or molding, or the alignment 75 of the platelets may be done in a separate process step.

One or more portions of this process 50 for forming an inflatable balloon may be similar to that used to form a conventional balloon. More specifically, short tubes can be made by extrusion with optional subsequent cutting of the tubes. A small parison may be formed by heating two segments of the tube and then drawing it. The heated segments are separated by a few millimeters to a few centimeters depending on desired parison size. The tube or the parison and most of the drawn tube is placed in a mold capable of applying heating, stretching and internal pressure to the balloon. These steps can be done stepwise or in combination in a defined sequence. The stretching in the mold can impart axial orientation of platelet nanofillers and the pressure (inflation) of the parison may impart circumferential orientation. After a balloon has been formed in the mold it is usually heatset (annealed) for a period of time and then cooled.

Still referring to Figure 5, the compounding of the polymeric matrix may be accomplished in a way that simplifies or reduces the difficulty of dispersing the platelet nanofillers into the polymeric material. According to one aspect of the present disclosure the goal for dispersing 80 the platelet nanofillers into the polymeric matrix is to achieve a homogeneous dispersion of the platelet nanofillers throughout the polymeric matrix.

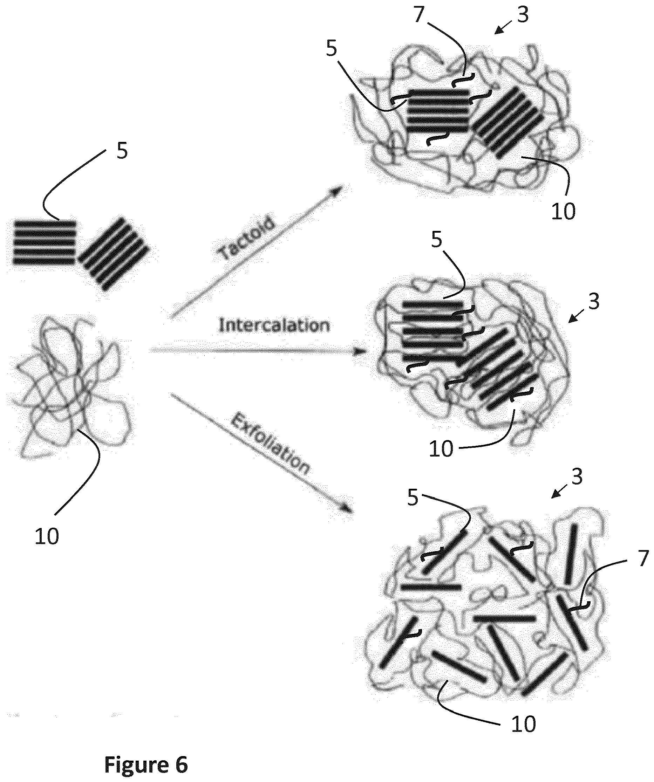

Since the platelet nanofillers comprise a layered material, the dispersion of the platelet nanofillers may involve tactoid dispersion, substantial intercalation or alternatively, exfoliation. The occurrence of either intercalation or exfoliation is desired, while the presence of a substantial amount of tactoid formation should be avoided if possible. Referring now to Figure 6, the mixing of the platelet nanofillers 5 with the polymeric matrix 10 may lead to the formation of a nanoplatelet composite material 3 that comprises small clusters of the platelet nanofillers 5 dispersed in the polymeric matrix. As used herein interacalation refers to the reversible inclusion or insertion of a molecule or ion into a material that exhibits a layered structure. Exfoliation represents an extreme case of intercalation in which complete separation of the layers present in the material occurs.

Still referring to Figure 6, the nanoplatelet composite material 3 comprises a dispersion of platelet nanofillers 5 in a polymeric matrix 10. The platelet nanofillers 5 from a top-down view are layered materials that resemble the geometric form of rectangles, squares, hexagons, and/or other polygons extending in two dimensions (length - width). In Figure 6, the platelet nanofillers 5 are shown from the perspective of their third dimension, thickness, in which the platelets are extremely thin or short. In this perspective view, the platelets extend "inwards" into the paper to provide the dimensions of length and width. During dispersion and alignment the platelets extend in the axial and circumferential directions relative to the length of the inflatable balloon in which the nanoplatelet composite material 3 is incorporated as opposed to the axial and radial directions or the radial and circumferential directions. When desirable, in order to enhance dispersion the nanoplatelet composite material 3 may also comprise one or more dispersants or surfactants 7 as shown in Figure 6 to modify the hydrophilic and/or hydrophobic characteristics of the platelets 5. Optionally, other additives may be included in the nanoplatelet composite materials 5 as separate materials or organic modification of the platelets' surface in order to enhance the interaction of the platelets 5 with the polymeric matrix 10.

Since the platelet nanofillers are layered materials, the various layers can slide with respect to one another during dispersion into the polymeric matrix. Such a structure is more responsive to tension in the parallel direction than in the perpendicular one. In addition to the orientation of the platelets influencing the behavior of the nanoplatelet composite material, the behavior of the composite material can also be affected by the amount of platelets that are dispersed into the matrix and the degree to which separation occurs between the layers in the platelets (e.g., occurrence of intercalation or exfoliation) during dispersion.

Referring once again to Figure 5, the dispersion 65 of the platelet nanofillers into the polymeric matrix may be a separate step conducted in the manufacturing process 50 or incorporated and performed as part of the polymerization process 57 associated with forming the polymeric matrix material. The platelet nanofillers may be dispersed 80 into the polymeric matrix by mixing the two components together using a micronizer or homogenizer, a mechanical stirrer, a mill, an ultrasonic stirrer, a magnetic stirrer, or the like. This dispersion may include the formation 83 of dispersed clusters of the platelet nanofillers (tactoid), partial separation of the platelets in the dispersed nanofillers (intercalatiaon), or substantial or complete separation of the platelets in the dispersed nanofillers (exfoliation). Alternatively, the separation of the platelets or layers in the platelet nanofillers occurs during the dispersion of the nanofillers into the polymeric matrix.

The balloon forming process 70 can be accomplished using conventional methods including, but not limited to, an extrusion process, a blow molding process, an injection molding process, a compression molding process, a sheet-forming process, or the like. The use of an extrusion process may promote isotropy (i.e., same properties in all directions) in the inflatable balloon. Alternatively, axial orientation of the platelet nanofillers in the polymeric matrix may also be promoted by the use of an extrusion process.

Optionally, the reinforced nanocomposite material may include a coating that is applied onto another polymer substrate that represents a bulk layer in the inflatable balloon. In this respect, the method 50 may further comprise providing 67 a layer of a polymer material having an external surface and applying 68 the nanocomposite material (i.e., the platelet nanofillers dispersed in the polymeric matrix) as a coating onto the external surface of the polymer layer.

The alignment 85 of the platelet nanofillers in the polymeric matrix may be accomplished using on or more of mechanical stretching of the nanocomposite; magnetic field induced alignment, shear force or friction induced alignment, AC electric field alignment, electrospinning, or electrophoretic alignment, to name a few.

The stretching of the nanoplatelet composite material refers to treatment of inflatable balloons 2 as sheets of the nanoplatelet composite materials 3 and pulling or applying mechanical loads to the sheets in opposed or offset directions. The stretching of the nanoplatelet composite material induces strain in the composite thereby forcing the platelet nanofillers to reorient themselves. For example, the sheets may be mechanically stretched by passing the sheets between a set of rollers at different speeds, such that one roller is set at a relatively slower rotational speed and "holds" the sheet, while the other roller is set at a relatively faster rotational speed and "stretches" the sheet. The degree of platelet nanofillers alignment may be adjusted by varying the degree of stretching and by varying the temperature at which the stretch takes place. When sheets are stretched, the stretched sheets can be formed into tubes by joining two opposing edges of a sheet.

In a shear force or friction induced alignment process, a nanoplatelet composite with embedded platelet nanofillers is first softened by warming. Then, the softened nanoplatelet composite is unidirectionally rubbed with a blade or a set of parallel grooves, which induces an elastic force that can align the nanotubes, but it also damages the nanocomposite. The advantage of this procedure is that it can be performed automatically.

The use of electric fields to align platelet nanofillers is a technique called electrophoresis. The application of an AC electric field aligns the individual platelet nanofillers between the electrodes. The use of a DC electric field is not suitable as it results in accumulation of platelet nanofillers near one electrode. The use of this technique is sensitive to a combination of several parameters including platelet nanofillers concentration and strength and frequency (kHz-MHz range) of the electric field. The heating of the polymeric matrix to a temperature that is above it's glass transition temperature ($T_g$) is done in order to allow the platelet nanofillers to rearrange themselves in the polymer matrix. Electrostatic repulsion between charged platelet nanofillers can enhance the alignment of the platelet nanofillers in the nanoplatelet composite materials.

Magnetic fields may also be utilized to align platelet nanofillers when the platelet nanofillers exhibit magnetic properties. The platelet nanofillers align in the direction of the magnetic field. Contrary to an electric field, which also cause the platelet nanofillers to move, a magnetic field will only reorient the platelet nanofillers.

In addition, the resulting flow of the platelet nanofillers dispersed in the polymeric matrx into a mold or through an extrusion die may cause the platelet nanofillers contained within the mixture to at least partially align themselves. Aligning the platelet nanofillers circumferentially and axially results in increased tensile strength, stiffness, and hardness, as well as decreasing brittleness. Injection molding, casting, and hot pressing are examples of processes that are capable of the flow inducement of short platelet nanofillers alignment in a nanocomposite.

For the purpose of this disclosure the terms "about" and "substantially" are used herein with respect to measurable values and ranges due to expected variations known to those skilled in the art (e.g., limitations and variability in measurements).

As used herein, the term "polymer" refers to a molecule having polymerized units of one or more species of monomer. The term "polymer" is understood to include both homopolymers and copolymers. The term "copolymer" refers to a polymer having polymerized units of two or more species of monomers, and is understood to include terpolymers. As used herein, reference to "a" polymer or other chemical compound refers one or more molecules of the polymer or chemical compound, rather than being limited to a single molecule of the polymer or chemical compound. Furthermore, the one or more molecules may or may not be identical, so long as they fall under the category of the chemical compound. Thus, for example, "a" polyurethane is interpreted to include one or more polymer molecules of the polyurethane, where the polymer molecules may or may not be identical (e.g., different molecular weights).

For the purpose of this disclosure, the terms "at least one" and "one or more of an element are used interchangeably and may have the same meaning. These terms, which refer to the inclusion of a single element or a plurality of the elements, may also be represented by the suffix "(s)"at the end of the element. For example, "at least one polyurethane", "one or more polyurethanes", and "polyurethane(s)" may be used interchangeably and are intended to have the same meaning.

Those skilled-in-the-art, in light of the present disclosure, will appreciate that many changes can be made in the specific embodiments which are disclosed herein and still obtain alike or similar result without departing from or exceeding the spirit or scope of the disclosure. One skilled in the art will further understand that any properties reported herein represent properties that are routinely measured and can be obtained by multiple different methods. The methods described herein represent one such method and other methods may be utilized without exceeding the scope of the present disclosure.

Within this specification, embodiments have been described in a way which enables a clear and concise specification to be written, but it is intended and will be appreciated that embodiments may be variously combined or separated without parting from the invention. For example, it will be appreciated that all preferred features described herein are applicable to all aspects of the invention described herein.

The foregoing description of various forms of the invention has been presented for purposes of illustration and description. It is not intended to be exhaustive or to limit the invention to the precise forms disclosed. Numerous modifications or variations are possible in light of the above teachings. The forms discussed were chosen and described to provide the best illustration of the principles of the invention and its practical application to thereby enable one of ordinary skill in the art to utilize the invention in various forms and with various modifications as are suited to the particular use contemplated. All such modifications and variations are within the scope of the invention as determined by the appended claims when interpreted in accordance with the breadth to which they are fairly, legally, and equitably entitled.

## Claims

1. A medical device comprising:
   an inflatable balloon having an axial length with a wall located along the axial length having an interior surface and an exterior surface that defines a wall thickness there between; at least a portion of the wall being formed of a nanoplatelet composite material, the nanoplatelet composite material comprising:

a polymeric matrix; and
one or more platelet nanofillers dispersed therein and aligned in a predetermined pattern, wherein the predetermined pattern comprises the platelet nanofilers extending in at least two directions ranging from the platelet nanofillers being circumferentially-oriented to axially-oriented relative to the axial length of the inflatable balloon.

2. The medical device according to claim 2, wherein the inflatable balloon further comprises a layer of a polymer material that has an external surface, such that the nanoplatelet composite material is a coating layer located on the external surface of the layer of polymer material.

3. The medical device according to any of claims 1 or 2, wherein the platelet nanofillers are individually selected to comprise; graphite, graphene, minerals, and clays;
wherein the amount of platelet nanofillers present in the nanoplatelet composite material ranges from about 0.1 wt. % to less than 5 wt. %, based on the overall weight of the nanoplatelet composite material.

4. The medical device according to any of claims 1-3, wherein the platelet nanofillers are organically-modified with functional groups that are chemically compatible with the polymeric matrix and/or subjected to the exchange of sodium or potassium ions for alkylammonium ions or functionalized organosilanes.

5. The medical device according to any of claims 1-4, wherein the polymeric matrix comprises cellulose, polyamide, polyethylene terephthalate (PET), polybutylene terephthalate (PBT), polyethylene naphthalate (PEN), and ethylene terephthalate copolymers; polyurethane; polyethylene; polyvinyl chloride; polycarbonate; poly(meth)acrylate; maleate; polyether etherketone (PEEK); poly(ethylene-co-methacrylic acid) (EMAA); polyamide/polyether block copolymer, polyester/polyether block copolymer; polyamide/polyether polyester copolymer; PBT polybutylene oxide block copolymer or a combination thereof.

6. The medical device according to any of claims 1-5, wherein the inflatable balloon comprises two or more layers of the nanoplatelet composite material;
wherein the platelet nanofillers present in one layer are oriented in a different direction than the platelet nanofillers in another layer.

7. The medical device according to any of claims 1-6, wherein the platelet nanofillers are aligned, such that he platelet nanofillers are oriented biaxially in a direction that ranges between the platelet nanofillers being axially-oriented and circumferentially-oriented with respect to the axial length of the inflatable balloon.

8. The medical device according to any of Claims 1-7, wherein the inflatable balloon further comprises at least one of an inner protective layer and an outer protective layer.

9. The medical device according to any of claims 1-8, wherein the platelet nanofillers have a thickness in the range from about 5 Angstrom (A) to about 500 nanometers (nm) and a width or length that individually ranges from about 1 nanometer (nm) up to about 2 micrometers ($\mu$m), preferably wherein the platelet nanofillers exhibit an aspect ratio that ranges from 50 to about 5,000.

10. The medical device according to anyone of claims 1-9, wherein:

the majority or substantially all of the platelet nanofillers are oriented in planes orthogonal to the direction of wall thickness; and/or
the platelet nanofillers in a portion of the wall extending 360° about the axial length of the balloon are oriented predominately with a common orientation direction or oriented predominately with one of only two common orientation directions, for that portion.

11. A method of forming a medical device comprising an inflatable balloon having an axial length with a wall located along the axial length having an interior surface and an exterior surface that defines a wall thickness there between, the method comprising:

providing an polymeric matrix;

providing a one or more platelet nanofillers;

dispersing the one or more platelet nanofillers into the polymeric matrix to form a nanoplatelet composite material;

forming the inflatable balloon from the nanoplatelet composite material using an extrusion process, a blow molding process, an injection molding process, a compression molding process, or a sheet-forming process; and

aligning the platelet nanofillers in a predetermined pattern, wherein the predetermined pattern comprises the platelet nanofilers extending in at least two directions ranging from the platelet nanofillers being circumferentially-oriented to axially-oriented relative to the axial length of the inflatable balloon.

12. The method according to Claim 11, wherein the alignment of the platelet nanofillers is accomplished using on or more of mechanical stretching of the nanoplatelet composite; magnetic field induced alignment, shear force or friction induced alignment, AC electric field alignment, electrospinning, and electrophoretic alignment.

13. The method according to any of claims 11 or 12, wherein dispersing the one or more platelet nanofillers into the polymeric matrix comprises includes the occurrence of one or more of intercalation and exfoliation;

wherein, optionally, the platelet nanofillers are organically-modified with functional groups that are chemically compatible with the polymeric matrix and/or subjected to the exchange of sodium or potassium ions for alkylammonium ions or functionalized organosilanes, such that intercalation, exfoliation, or combination thereof occurs.

14. The method according to any of claims 11-13, wherein the method further comprises providing a layer of a polymer material having an external surface; and applying the nanoplatelet composite material as a coating layer onto the external layer of the polymer material.

15. The use of a nanoplatelet composite material to form a medical device that includes an inflatable balloon having an axial length with a wall located along the axial length having an interior surface and an exterior surface that defines a wall thickness there between; at least a portion of the wall being formed of the nanoplatelet composite material, the nanoplatelet composite material comprising:

a polymeric matrix; and

one or more platelet nanofillers aligned in a predetermined pattern, wherein the predetermined pattern comprises the platelet nanofilers extending in at least two directions ranging from the platelet nanofillers being circumferentially-oriented to axially-oriented relative to the axial length of the inflatable balloon.

**Figure 1**

**Figure 2**

2C 10C 5C a

x

a

**Figure 3A**
(Comparable)

5C 10C

x

2C

**Figure 3B**
(Comparable)

Figure 4A

Figure 4B

50

57

80

Dispersing nanofillers in polymeric matrix done using:
- a micronizer or homogenizer
- a mechanical stirrer
- a mill
- an ultrasonic stirrer, or
- a magnetic stirrer

| Providing an polymeric matrix | Providing one or more platelet nanofilers |

55    60

83

Formation of nanoplatelet composite with
- *Tactoid dispersion*
- *Intercalation*
- *Exfoliation*

Dispersing the platelet nanofillers into the polymeric matrix to form a nanoplatelet composite

65

optional

67

Providing a layer of a polymer material

68

Forming inflatable balloon from nanoplatelet composite using:
- *Extrusion process*
- *Blow molding process*
- *Injection molding process*
- *Compression molding process; or*
- *Sheet-forming process*

70

Applying the nanocomposite as a coating onto the external surface of the polymer material

75

Aligning platelet nanofillers circumferentially or axially

Alignment done using:
- *Mechanical stretching*
- *magnetic field induced alignment*
- *shear force or friction induced alignment*
- *AC electric field alignment*
- *electrospinning, or*
- *electrophoretic alignment*

85

**Figure 5**

Figure 6

Europäisches
Patentamt

European
Patent Office

Office européen
des brevets

## EUROPEAN SEARCH REPORT

**Application Number**

EP 18 18 9989

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/143350 A1 (JIMENEZ OSCAR [US]) 31 July 2003 (2003-07-31) * paragraph [0027] - paragraphs [0034], [0037]; claims * | 1-15 | INV. A61L29/12 A61L29/08 A61F2/958 A61M25/10 B82Y30/00 |
| X | EP 1 388 346 A1 (TERUMO CORP [JP]) 11 February 2004 (2004-02-11) * paragraphs [0014], [0048], [0100], [0108]; examples 4-7 * | 1-15 | |
| X | EP 1 858 441 B1 (BOSTON SCIENT LTD [BB]) 11 April 2012 (2012-04-11) * paragraphs [0004], [0006], [0011], [0012], [0015] - [0017], [0019], [0030], [0040] * | 1-15 | |
| X | WO 2005/112845 A1 (BOSTON SCIENT SCIMED INC [US]; WEBER JAN [US]; HOKANSON JOHN MICHAEL [US]) 1 December 2005 (2005-12-01) * page 5, line 15 - line 27 * * page 9, line 16 - line 24 * * page 10, line 3 - line 16; claims * | 1-15 | |
| A | WO 2005/042630 A2 (HUNTSMAN ADV MAT SWITZERLAND [CH]; MILLER MICHELLE [GB]) 12 May 2005 (2005-05-12) * page 8, line 2 - line 18 * * page 11, line 31 - page 13, line 8 * | 1-15 | TECHNICAL FIELDS SEARCHED (IPC)  A61L A61F A61M B82Y |
| A | WO 2012/151433 A2 (UNIV OKLAHOMA STATE [US]; HANAN JAY CLARKE [US]) 8 November 2012 (2012-11-08) * page 3, line 23 - line 30; claims; examples * | 1-15 | |

The present search report has been drawn up for all claims

| Place of search | Date of completion of the search | Examiner |
|---|---|---|
| The Hague | 21 January 2019 | Espinosa y Carretero |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 18 9989

This annex lists the patent family members relating to the patent documents cited in the above-mentioned European search report.
The members are as contained in the European Patent Office EDP file on
The European Patent Office is in no way liable for these particulars which are merely given for the purpose of information.

21-01-2019

| Patent document cited in search report | | Publication date | Patent family member(s) | | Publication date |
|---|---|---|---|---|---|
| US 2003143350 | A1 | 31-07-2003 | US | 7419633 B1 | 02-09-2008 |
| | | | US | 2003143350 A1 | 31-07-2003 |
| | | | US | 2008103444 A1 | 01-05-2008 |
| | | | US | 2011087262 A1 | 14-04-2011 |
| | | | US | 2013317536 A1 | 28-11-2013 |
| EP 1388346 | A1 | 11-02-2004 | AT | 323518 T | 15-05-2006 |
| | | | DE | 60304633 T2 | 05-04-2007 |
| | | | EP | 1388346 A1 | 11-02-2004 |
| | | | JP | 4351832 B2 | 28-10-2009 |
| | | | JP | 2004065413 A | 04-03-2004 |
| | | | US | 2004101644 A1 | 27-05-2004 |
| EP 1858441 | B1 | 11-04-2012 | AT | 552802 T | 15-04-2012 |
| | | | CA | 2597597 A1 | 24-08-2006 |
| | | | EP | 1858441 A1 | 28-11-2007 |
| | | | JP | 5185630 B2 | 17-04-2013 |
| | | | JP | 2008529733 A | 07-08-2008 |
| | | | US | 2006184112 A1 | 17-08-2006 |
| | | | US | 2012046608 A1 | 23-02-2012 |
| | | | WO | 2006088989 A1 | 24-08-2006 |
| WO 2005112845 | A1 | 01-12-2005 | AT | 447386 T | 15-11-2009 |
| | | | CA | 2566686 A1 | 01-12-2005 |
| | | | EP | 1750631 A1 | 14-02-2007 |
| | | | ES | 2336013 T3 | 07-04-2010 |
| | | | JP | 4991542 B2 | 01-08-2012 |
| | | | JP | 2007537839 A | 27-12-2007 |
| | | | US | 2005261670 A1 | 24-11-2005 |
| | | | US | 2010286678 A1 | 11-11-2010 |
| | | | US | 2013123832 A1 | 16-05-2013 |
| | | | WO | 2005112845 A1 | 01-12-2005 |
| WO 2005042630 | A2 | 12-05-2005 | BR | PI0416166 A | 09-01-2007 |
| | | | CA | 2540893 A1 | 12-05-2005 |
| | | | CN | 1875056 A | 06-12-2006 |
| | | | EP | 1682608 A2 | 26-07-2006 |
| | | | JP | 4799416 B2 | 26-10-2011 |
| | | | JP | 2007514009 A | 31-05-2007 |
| | | | US | 2007072981 A1 | 29-03-2007 |
| | | | US | 2011301264 A1 | 08-12-2011 |
| | | | WO | 2005042630 A2 | 12-05-2005 |
| WO 2012151433 | A2 | 08-11-2012 | AU | 2012250670 A1 | 19-12-2013 |
| | | | BR | 112013028400 A2 | 24-01-2017 |
| | | | CA | 2835112 A1 | 08-11-2012 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 18 18 9989

This annex lists the patent family members relating to the patent documents cited in the above-mentioned European search report.
The members are as contained in the European Patent Office EDP file on
The European Patent Office is in no way liable for these particulars which are merely given for the purpose of information.

21-01-2019

| Patent document cited in search report | Publication date | Patent family member(s) | | Publication date |
|---|---|---|---|---|
| | | CN | 103814080 A | 21-05-2014 |
| | | CO | 6811879 A2 | 16-12-2013 |
| | | EP | 2705091 A2 | 12-03-2014 |
| | | EP | 3372644 A1 | 12-09-2018 |
| | | ES | 2673289 T3 | 21-06-2018 |
| | | IL | 229216 A | 31-01-2017 |
| | | JP | 6113148 B2 | 12-04-2017 |
| | | JP | 2014513186 A | 29-05-2014 |
| | | KR | 20140034802 A | 20-03-2014 |
| | | KR | 20180043400 A | 27-04-2018 |
| | | MX | 359481 B | 28-09-2018 |
| | | RU | 2013153379 A | 10-06-2015 |
| | | US | 2014080962 A1 | 20-03-2014 |
| | | US | 2017218166 A1 | 03-08-2017 |
| | | US | 2019010304 A1 | 10-01-2019 |
| | | WO | 2012151433 A2 | 08-11-2012 |

EPO FORM P0459

For more details about this annex : see Official Journal of the European Patent Office, No. 12/82

page 2 of 2